# EUROPÄISCHE PATENTSCHRIFT

(11) **EP 1 895 325 B1**
(45) Veröffentlichungstag und Bekanntmachung des Hinweises auf die Patenterteilung: **31.03.2010**
(21) Anmeldenummer: 06119938.6
(22) Anmeldetag: 31.08.2006
(51) Int. Cl.: G01T 1/29

(54) **Löschen einer Speicherleuchtstoffschicht**
Erasing of a phosphor imaging plate
Effacement d'une couche d'enregistrement luminescente

(43) Veröffentlichungstag der Anmeldung: 05.03.2008
(73) Patentinhaber: Agfa HealthCare NV, 2640 Mortsel (BE)
(72) Erfinder: Bode, Andreas, Dr., 81476 München (DE); Reiser, Georg, Dr., 80337 München (DE); Weber, Marc, Dr., 81679 München (DE); Thoma, Ralph, Dr., 86167 Augsburg (DE); Stallmeister, Stefan, 85625 Glonn (DE); Baunach, Stephan, 80339 München (DE); Minwegen, Heike, 83043 Bad Aibling (DE); Dedler, Rupert, 81547 München (DE); Fasbender, Robert, Dr., 91054 Erlangen (DE)
(74) Vertreter: Linsmeier, Josef

(56) Entgegenhaltungen:
- EP-A1- 0 704 716
- EP-A2- 0 964 270
- US-A1- 2003 123 613
- US-A1- 2005 012 057
- US-A1- 2005 017 207
- US-B1- 6 528 812

## Beschreibung

Die vorliegende Erfindung betrifft eine Vorrichtung zum Löschen einer Speicherleuchtstoffschicht mit einer Strahlungsquelle zum Erzeugen und Aussenden einer Löschstrahlung, einem Antrieb zum Erzeugen einer Relativbewegung zwischen der Speicherleuchtstoffschicht und der Strahlungsquelle, wobei die Speicherleuchtstoffschicht in einer Aufnahmeebene liegt bzw. bewegt wird, und einem Reflektor zum Reflektieren von Strahlung. Die vorliegende Erfindung betrifft ferner ein System mit einer solchen Vorrichtung und einer Speicherleuchtstoffschicht.

Eine solche Vorrichtung und ein solches System werden insbesondere im Bereich der Computer-Radiografie (CR) für medizinische Zwecke eingesetzt. Von einem Objekt, beispielsweise einem Patienten oder einem Körperteil des Patienten, wird mittels Röntgenbestrahlung ein Bild erzeugt, das in einer Speicherleuchtstoffschicht als latentes Bild abgespeichert wird. Ein solches Röntgenbild enthält somit Röntgeninformationen über das Objekt. Zum Auslesen der in der Speicherleuchtstoffschicht abgespeicherten Röntgeninformationen wird die Speicherleuchtstoffschicht mittels einer Bestrahlungseinrichtung angeregt. Die Speicherleuchtstoffschicht emittiert aufgrund dieser Anregung Strahlung, die eine Intensität entsprechend der in der Speicherleuchtstoffschicht abgespeicherten Röntgeninformationen aufweist. Die von der Spolcherleuchtstoffschicht ausgesandte Strahlung wird von einer Detektionseinrichtung erfasst und in elektrische Signale gewandelt, die ein Abbild der Röntgeninformationen enthalten. Die elektrischen Signale werden weiterverarbeitet und die in der Speicherleuchtstoffschicht abgespeicherten Röntgeninformationen anschließend sichtbar gemacht. Die Röntgeninformationen können beispielsweise direkt auf einem Monitor dargestellt oder mittels eines speziell für Röntgenbilder verwendbaren Druckers auf einen fotografischen Röntgenfilm geschrieben werden.

Nach dem Auslesen der Röntgeninformationen aus der Speicherleuchtstoffschicht verbleiben in dieser Reste des latenten Bildes. Ferner können Rauschinformationen abgespeichert sein. Um die Speicherleuchtstoffschicht für weitere Röntgenaufnahmen verwenden zu können, wird sie daher gelöscht. Dazu wird eine Strahlungsquelle verwendet, die eine Löschstrahlung auf die Speicherleuchtstoffschicht ausgibt. Aus der US 7,057,200 B2 ist eine Vorrichtung zum Löschen einer Speicherleuchtstoffschicht bekannt. Diese Löschvorrichtung enthält als Strahlungsquelle zum Aussenden der Löschstrahlung zwei parallel zueinander angeordnete Zeilen mit Leuchtdioden. Zum Löschen wird die Speicherleuchtstoffschicht in eine Transportrichtung durch einen Strahlengang der Leuchtdiodenzeilen geschoben. Die beiden Leuchtdiodenzeilen sind in Reflektorren integriert, die voneinander beabstandet sind. Die Reflektoren dienen dazu, von den Leuchtdioden ausgegebene Löschstrahlung in Richtung der Speicherleuchtstoffschicht zu reflektieren. Die Reflektoren haben eine rinnenförmige Querschnittsfläche mit stumpfen Innenwinkeln, so dass sich das rinnenförmige Profil der Reflektoren ausgehend von der Anordnung der Leuchtdioden in Richtung der Speicherleuchtstoffschicht öffnet. Dabei ist allerdings die Ausbreitung der Reflektoren in Transportrichtung an ihre Funktion der Strahlungsführung der von den Leuchtdioden emittierten Löschstrahlung angepasst und in Relation zu dem Abstand der Reflektoren zu der Speicherleuchtstoffschicht relativ klein.

Aus US 2003/0123613 A1 ist eine Vorrichtung bekannt, bei welcher eine zu löschende Bildplatte auf einem gebogenen Weg durch eine teilzylinderförmige Löscheinheit geführt wird. Die äußere Umfangsfläche der Löscheinheit weist Lichtquellen auf und ist mit einer reflektierenden Beschichtung versehen, durch welche von der Bildplatte reflektiertes Löschlicht wieder zu dieser zurück reflektiert wird. Diese Vorrichtung wird als nächster Stand der Technik angesehen.

Es ist die Aufgabe der vorliegenden Erfindung, eine hohe Effizienz beim Löschen einer Speicherleuchtstoffschicht zu ermöglichen.

Diese Aufgabe wird gemäß der technischen Lehre des Anspruchs 1 oder des Anspruchs 17 gelöst. Vorteilhafte Ausgestaltungen der Erfindung können den abhängigen Ansprüchen entnommen werden.

Erfindungsgemäß ist der Reflektor zum Reflektieren von von der Speicherleuchtstoffschicht reflektierter Löschstrahlung in Richtung der Speicherleuchtstoffschicht angeordnet und ausgestaltet. Dazu ist eine Ausbreitung des Reflektors in Richtung der Relativbewegung wenigstens zehnmal so groß, wie ein kleinster Abstand des Reflektors von der Aufnahmeebene. Darüber hinaus weist der Reflektor eine rinnen- oder trogförmige Querschnittsfläche auf. Ein solcher Reflektor ist besonders einfach herstellbar und erreicht einen hohen Wirkungsgrad beim Löschen.

Der vorliegenden Erfindung liegt die Erkenntnis zugrunde, dass die Speicherleuchtstoffschicht üblicherweise einen hohen Reflexionsgrad hat. Dadurch wird ein großer Teil der Löschstrahlung ungenutzt reflektiert und trägt nicht zum Löschen von in der Speicherleuchtstoffschicht abgespeicherten, unerwünschten Bildinformationen bei. Aufgrund der vorliegenden Erfindung wird die von der Speicherleuchtstoffschicht reflektierte Löschstrahlung von dem Reflektor erneut in Richtung der Speicherleuchtstoffschicht zurückreflektiert. Diese Reflexion kann gerichtet oder diffus sein. Die von dem Reflektor zurückreflektierte Löschstrahlung kann somit ebenfalls zum Löschen der Speicherleuchtstoffschicht beitragen. Dadurch wird der Wirkungsgrad des Löschens wesentlich verbessert. Ferner ist der Leistungsbedarf geringer, was zu weniger Verlustwärme und zu einer Erhöhung der Lebensdauer führt. Des Weiteren ist davon auszugehen, dass die Ausfallrate reduziert wird. Gegebenenfalls kann dadurch auf ein Kühlen der Strahlungsquelle verzichtet werden. Dies gewährleistet eine kostengünstige Löschvorrichtung. Ferner wird vorteilhafterweise ein geringer Platzbedarf möglich. Die Ausgestaltung des Reflektors kann vorteilhafterweise auf einfache Weise an die Art und Weise und die Form der Speicherleuchtstoffschicht angepasst werden. Der Reflektor ist insbesondere so angeordnet, dass er wenigstens eine Reflexionsfläche enthält, die der Aufnahmeebene zugewandt ist. Durch die Dimensionierung des Reflektors mit seiner großen Ausbreitung in Richtung der Relativbewegung und seinem Abstand zu der Aufnahmeebene für die Speicherleuchtstoffschicht kann insbesondere gewährleistet werden, dass ein großer Teil der von der Speicherleuchtstoffschicht reflektierten oder gestreuten Löschstrahlung aufgefangen und erneut in Richtung der Speicherleuchtstoffschicht reflektiert werden kann.

In einer vorteilhaften Ausgestaltung der Erfindung weist der Reflektor eine ebene Reflektorfläche auf, die parallel zur Aufnahmeebene verläuft. Eine solche Reflektorform kann zuverlässig reflektierte Löschstrahlung zur Speicherleuchtstoffschicht zurückreflektieren. Diese Reflektorform ist kostengünstig herstellbar und kompakt ausgestaltbar.

In einer weiteren vorteilhaften Ausgestaltung weist der Reflektor eine Reflektorfläche mit einer Struktur auf. Mit einer solchen Struktur kann die Effizienz des Löschens noch weiter erhöht werden. Die Struktur kann insbesondere geriffelt, dach-, sägezahn- oder dreieckförmig, etc. sein.

In einer besonders bevorzugten Ausgestaltung der Erfindung ist die strukturierte Reflektorfläche retroreflektiv ausgestaltet, so dass sie zumindest einen Teil der von der Speicherleuchtstoffschicht zurückgestreuten Löschstrahlung an dieselben Stellen der Speicherleuchtstoffschicht zurückreflektiert. Eine solche retroreflektive Reflektorfläche gewährleistet ein besonders effizientes Löschen der Speicherleuchtstoffschicht. An diejenigen Stellen, die viel Löschstrahlung reflektiert haben, wird auch viel Löschstrahlung zurückreflektiert. Die retroreflektive Reflektorfläche kann insbesondere nach Art eines sogenannten "Katzenauges" ausgestaltet sein und beispielsweise als Folie eingesetzt werden. Dies ist besonders platzsparend und kostengünstig.

Besonders vorteilhaft weist der Reflektor wenigstens zwei Reflektorflächen auf, so dass der Reflektor, in Richtung der Relativbewegung betrachtet, zu beiden Seiten der Strahlungsquelle ausgebildet ist. Dadurch kann besonders viel Löschstrahlung erfasst und zurückreflektiert werden.

Die rinnen- oder trogförmige Querschnittsfläche weist stumpfe Innenwinkel auf, die insbesondere größer oder gleich 130° sind. Dadurch wird die Menge an reflektierter Löschstrahlung, die wieder zur Speicherleuchtstoffschicht zurückreflektiert wird, noch weiter erhöht.

Alternativ oder zusätzlich dazu schließen sich, in Richtung der Relativbewegung betrachtet, an die beiden Enden der Rinne parallel zur Aufnahmeebene verlaufende Reflektor-Fortsatzflächen an. Vorzugsweise weisen die Reflektor-Fortsatzflächen Strukturen auf. Diese Ausgestaltungen gewährleisten jeweils eine noch bessere Effizienz des Löschvorgangs.

In einer weiteren vorteilhaften Ausgestaltung weist der Reflektor an den der Aufnahmeebene zugewandten Reflektorflächen reflektierende Schichten auf. Dadurch kann der Reflexionsgrad merklich verbessert werden.

In einer besonders bevorzugten Ausgestaltung der Erfindung ist der Reflektor in Richtung der Relativbewegung spiegelsymmetrisch ausgestaltet, wobei eine Symmetrieachse senkrecht zur Richtung der Relativbewegung und, in Richtung der Relativbewegung betrachtet, zentral durch die Strahlungsquelle hindurch verläuft. Durch einen solchen Reflektor kann zu beiden Seiten der Strahlungsquelle eine große Menge an reflektierter Löschstrahlung erfasst und zur Speicherleuchtstoffschicht zurückreflektiert werden.

Besonders vorteilhaft weist die Strahlungsquelle wenigstens zwei senkrecht zur Richtung der Relativbewegung und parallel zu der Aufnahmeebene verlaufende Zeilen mit Leuchtdioden auf. Dadurch lässt sich eine ausreichend hohe Intensität von Löschstrahlung erzeugen, wobei der Leistungsverbrauch der Leuchtdioden besonders gering ist.

Bevorzugt sind die wenigstens zwei Zeilen mit Leuchtdioden in den Reflektor integriert. Der Abstand zwischen den wenigstens zwei Zeilen und/oder der Abstand zwischen den einzelnen Leuchtdioden innerhalb der jeweiligen Zeile ist kleiner oder gleich einem Abstand der Leuchtdioden zu der Aufnahmeebene. Der Reflektor, und damit auch die Löschvorrichtung, kann dadurch besonders kompakt ausgestaltet werden. Ferner wird die von den einzelnen Leuchtdioden emittierte Löschstrahlung besonders gut durchmischt auf die Speicherleuchtstoffschicht ausgegeben.

Besonders bevorzugt ist den wenigstens zwei Zeilen mit Leuchtdioden jeweils ein eigener Reflektor zugeordnet. Die Leuchtdioden der jeweiligen Zeilen emittieren ferner eine Strahlung in einem anderen, insbesondere schmalbandigen, Wellenlängenbereich, als die Leuchtdioden der anderen Zeilen. Die Reflektoren sind insbesondere so ausgestaltet, dass sie zu einer Trennung der von den unterschiedlichen Leuchtdiodenzeilen ausgegebenen Löschstrahlungen mit den unterschiedlichen Wellenlängenbereichen beitragen. Dadurch kann verhindert werden, dass sich bestimmte Spektralbereiche gegenseitig beeinflussen oder stören. Die Wellenlängenbereiche können vorteilhafterweise so gewählt sein, dass solche Wellenlängen, die nicht zum Löschen des eingesetzten Typs von Speicherleuchtstoffschicht beitragen, nicht vorhanden sind. Dadurch ist ein sonst erforderliches Herausfiltern solcher Wellenlängen nicht erforderlich. Ferner wird eine besonders gute Löscheffizienz erreicht.

Vorzugsweise sind die wenigstens zwei Zeilen mit Leuchtdioden in Richtung der Relativbewegung so hintereinander angeordnet, dass beim Ausführen der Relativbewegung zum Löschen der Speicherleuchtstoffschicht eine kurzwellige Löschstrahlung vor einer langwelligen Löschstrahlung auf die Speicherleuchtstoffschicht trifft. Dabei kann insbesondere blaue Löschstrahlung vor roter Löschstrahlung auf die Speicherleuchtstoffschicht gerichtet sein. Auf diese Weise ist eine besonders gute Löscheffizienz gewährleistet.

Bevorzugt ist eine Intensität der langwelligen Löschstrahlung größer, als eine Intensität der kurzwelligen Löschstrahlung. Insbesondere kann ein Verhältnis von blauer zu roter Löschstrahlung so gewählt sein, dass 66% der Löschstrahlung rote und 33% der Löschstrahlung blaue Löschstrahlung ist. Dies gewährleistet eine noch bessere Löscheffizienz.

Besonders bevorzugt ist eine dem Reflektor, in Richtung senkrecht zur Richtung der Relativbewegung betrachtet, gegenüberliegende, weitere reflektierende Fläche zum Reflektieren von Löschstrahlung vorhanden. Die weitere reflektierende Fläche kann so ausgestaltet sein, dass sie die Löschstrahlung gerichtet oder diffus reflektiert. Durch diese weitere reflektierende Fläche kann vorteilhafterweise zu Beginn und/oder am Ende des Löschvorgangs, d. h. wenn der Strahlengang der Strahlenquelle nicht oder nicht vollständig auf die Speicherleuchtstoffschicht gerichtet ist, die von der Strahlungsquelle ausgegebene Löschstrahlung von der weiteren reflektierenden Fläche in Richtung des Reflektors reflektiert werden. Die von der weiteren reflektierenden Fläche reflektierte Löschstrahlung, die die Speicherleuchtstoffschicht insbesondere noch nicht erreicht hat, kann somit von dem Reflektor auf die Speicherleuchtstoffschicht gerichtet werden. Insbesondere die vordere und ggf. die hintere Kante der Speicherleuchtstoffschicht können somit mit hoher Effizienz gelöscht werden.

Vorzugsweise ist die weitere reflektierende Fläche auf der dem Reflektor abgewandten Seite der Aufnahmeebene angeordnet. Dadurch kann besonders gut gewährleistet werden, dass nicht auf die Speicherleuchtstoffschicht auftreffende Löschstrahlung von der weiteren reflektierenden Fläche reflektiert wird, um dann von dem Reflektor in Richtung der Speicherleuchtstoffschicht reflektiert zu werden.

Besonders bevorzugt weist die weitere reflektierende Fläche in Richtung der Relativbewegung eine Ausbreitung auf, die zumindest so groß ist, wie diejenige des Reflektors. Dadurch kann gewährleistet werden, dass Formen der weiteren reflektierenden Fläche und des Reflektors besonders gut aufeinander abgestimmt sind. Eine besonders große Menge von von der Strahlungsquelle emittierter Löschstrahlung kann von der weiteren reflektierenden Fläche reflektiert und eine große Menge dieser reflektierten Löschstrahlung von dem Reflektor in Richtung der Speicherleuchtstoffschicht reflektiert werden. Dadurch lässt sich eine besonders gute Löscheffizienz erreichen.

Vorzugsweise weist die Speicherleuchtstoffschicht des erfindungsgemäßen Systems einen Reflexionsgrad für die Löschstrahlung von größer oder gleich 70%, insbesondere größer oder gleich 80%, auf. Gerade für Speicherleuchtstoffschichten mit einem solch hohen Reflexionsgrad kann die erfindungsgemäße Löschvorrichtung besonders effizient eingesetzt werden.

Weitere Vorteile der Erfindung ergeben sich aus der folgenden Beschreibung von Ausführungsbeispielen, wobei Bezug auf die beigefügten Zeichnungen genommen wird. Es zeigen:
- Fig. 1: ein erstes Ausführungsbeispiel einer Löschvorrichtung mit einem Reflektor, der ebene, parallel zu einer Speicherleuchtstoffschicht verlaufende Reflektorflächen aufweist,
- Fig. 2: ein zweites Ausführungsbeispiel einer Löschvorrichtung mit einem Reflektor, der parallel zu der Speicherleuchtstoffschicht verlaufende Reflektorflächen mit einer dreieckförmigen Struktur aufweist,
- Fig. 3: ein drittes Ausführungsbeispiel einer Löschvorrichtung mit einem rinnenförmigen Reflektor,
- Fig. 4: ein viertes Ausführungsbeispiel einer Löschvorrichtung mit einem rinnenförmigen Reflektor, der ausgehend von den Enden der Rinne seitliche Reflektor-Fortsätze aufweist, die sich eben und parallel zu der Speicherleuchtstoffschicht erstrecken,
- Fig. 5: ein fünftes Ausführungsbeispiel einer Löschvorrichtung mit dem rinnenförmigen Reflektor, der ausgehend von den Enden der Rinne seitliche Reflektor-Fortsätze aufweist, die sich parallel zu der Speicherleuchtstoffschicht erstrecken und eine feine dreieckförmige Struktur aufweisen,
- Fig. 6: ein sechstes Ausführungsbeispiel einer Löschvorrichtung mit dem rinnenförmigen Reflektor, der ausgehend von den Enden der Rinne seitliche Reflektor-Fortsätze aufweist, die sich parallel zu der Speicherleuchtstoffschicht erstrecken und eine grobe dreieckförmige Struktur aufweisen,
- Fig. 7: ein siebtes Ausführungsbeispiel einer Löschvorrichtung mit einem Reflektor, der ausgehend von den Enden einer kleinen Rinne seitliche Reflektor-Fortsätze aufweist, die sich parallel zu der Speicherleuchtstoffschicht erstrecken und eine sägezahnförmige Struktur aufweisen,
- Fig. 8: ein achtes Ausführungsbeispiel einer Löschvorrichtung mit zwei Leuchtdiodenzeilen, die Licht in verschiedenen Wellenlängenbereichen ausgeben und jeweils einen eigenen Reflektor aufweisen, und
- Fig. 9: die Löschvorrichtung gemäß dem dritten Ausführungsbeispiel mit einer dem Reflektor gegenüberliegenden weiteren Reflexionsfläche.

Die Ausführungsbeispiele drei bis acht sind erfindungsgemäß.

Im Folgenden werden, sofern nicht anders angegeben, für gleiche oder gleichwirkende Elemente gleiche Bezugszeichen verwendet.

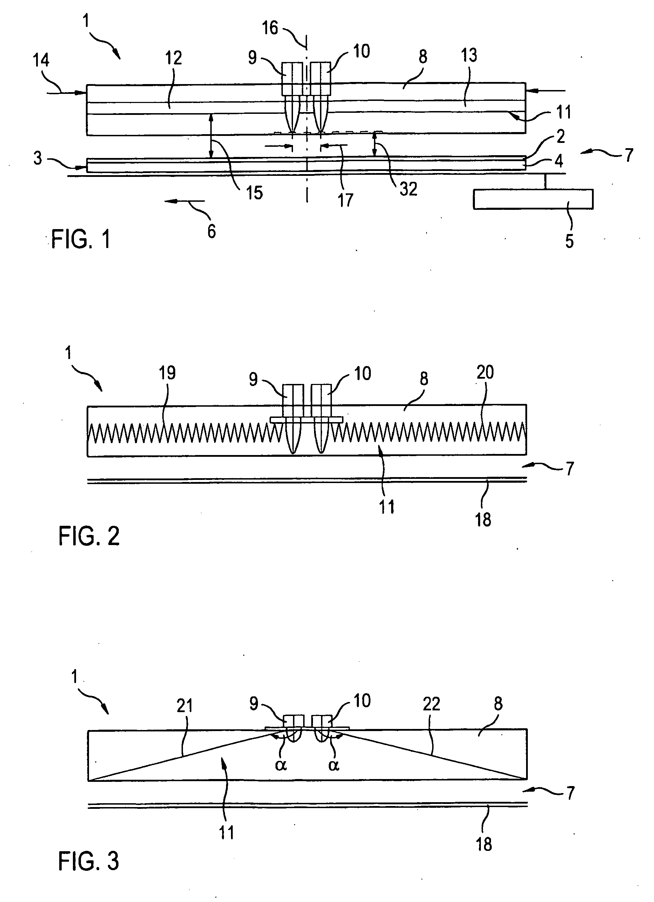

Fig. 1 zeigt ein erstes Ausführungsbeispiel einer Löschvorrichtung 1 zum Löschen von Röntgeninformationen, die in einer Speicherleuchtstoffschicht 2 einer Speicherleuchtstoffplatte 3 abgespeichert sind. Die Speicherleuchtstoffplatte 3 weist eine Trägerschicht 4 auf, auf der die Speicherleuchtstoffschicht 2 aufgebracht ist. Die Speicherleuchtstoffschicht 2 setzt sich vorzugsweise aus einer Vielzahl von Phophorpartikeln zusammen, die zum Speichern der Röntgeninformationen dienen. Die Trägerschicht 4 ist hier ein Laminat, das vorteilhafterweise eine Dicke von 1-2 mm hat. Die Speicherleuchtstoffplatte 3 ist hier nicht Teil der Löschvorrichtung 1, sondern kann von außen in die Löschvorrichtung 1 hineingeschoben werden. Innerhalb der Löschvorrichtung 1 wird die Speicherleuchtstoffplatte 3 mittels eines Antriebs 5 in eine Transportrichtung verschoben, die durch einen Pfeil 6 repräsentiert wird. Die Speicherleuchtstoffplatte 3 ist in der Löschvorrichtung 1 in einer Aufnahmeebene 7 geführt und in dieser Aufnahmeebene 7 verschiebbar.

Die Löschvorrichtung 1 enthält eine Strahlungsquelle 8 zum Aussenden von Löschstrahlung. Die Strahlungsquelle 8 weist hier zwei parallel zueinander angeordnete Leuchtdiodenzeilen 9 und 10 auf. Die Leuchtdiodenzeilen 9, 10 enthalten jeweils eine Vielzahl von nebeneinander angeordneten Leuchtdioden.

Die Leuchtdiodenzeilen 9, 10 erstrecken sich über die gesamte Länge der Speicherleuchtstoffschicht 2. Die Länge der Speicherleuchtstoffschicht 2 verläuft in der Darstellung nach Fig. 1 senkrecht zur Transportrichtung 6 und in Richtung der Zeichnungsblattebene. Die Breite der Speicherleuchtstoffschicht 2 erstreckt sich in Transportrichtung 6. Mittels des Antriebs 5 wird die Speicherleuchtstoffschicht 2 in Transportrichtung 6 mit gleichförmiger Transportgeschwindigkeit an den Leuchtdiodenzeilen 9, 10 vorbeitransportiert. Dadurch durchläuft die Speicherleuchtstoffschicht 2 die Strahlengänge der Leuchtdiodenzeilen 9, 10. Es ist alternativ auch möglich, anstelle der Speicherleuchtstoffplatte 3 die Strahlungsquelle zu transportieren, wobei dann die Speicherleuchtstoffplatte 3 in der Löschvorrichtung 1 nicht bewegt wird. In beiden Fällen wird zwischen der Strahlungsquelle 8 und der in der Aufnahmeebene 7 liegenden Speicherleuchtstoffschicht 2 eine Relativbewegung ausgeführt, die hier in Richtung des Pfeils 6 verläuft.

Beim Transportieren der Speicherleuchtstoffplatte 3 trifft das von den Leuchtdioden der Leuchtdiodenzeilen 9, 10 emittierte Löschlicht auf die Speicherleuchtstoffschicht 2. Ein Teil des Löschlichts dringt in die Speicherleuchtstoffschicht 2 ein und löscht die in ihr nach einem Auslesen verbliebenen Röntgeninformationen und ein gegebenenfalls vorhandenes Rauschen. Da die Speicherleuchtstoffschicht 2 für das Löschlicht einen Reflexionsgrad von wenigstens 70%, insbesondere von wenigstens 80%, aufweist, wird ein großer Teil des Löschlichts von der Speicherleuchtstoffschicht 2 reflektiert, ohne zum Löschen beizutragen.

Um eine hohe Effizienz und einen hohen Wirkungsgrad beim Löschen zu erzielen, hat die Löschvorrichtung 1 erfindungsgemäß einen Reflektor 11. Der Reflektor 11 weist im vorliegenden Ausführungsbeispiel zwei ebene, parallel zu der Speicherleuchtstoffschicht 2 verlaufende Reflektorflächen 12 und 13 auf. Die Reflektorflächen 12, 13 sind, in Transportrichtung 6 betrachtet, zu beiden Seiten der Strahlungsquelle 8 angeordnet und vorteilhafterweise gleich groß. Es ist aber ebenso möglich, nur eine einzige Reflektorfläche auf einer der Seiten der Strahlungsquelle 8 vorzusehen. Ferner ist es möglich, die Reflektorflächen 12, 13 einteilig oder eine der beiden kleiner auszugestalten, als die andere. Der Reflektor 11 erstreckt sich über die gesamte Länge der Speicherleuchtstoffschicht 2 und, in Transportrichtung 6 betrachtet, über eine Breite 14. Von der Oberfläche der in der Aufnahmeebene 7 befindlichen Speicherleuchtstoffschicht 2 weist der Reflektor einen kleinsten Abstand 15 auf. Die Breite 14 ist wenigstens zehnmal so groß, wie der kleinste Abstand 15. Der Reflektor 11 ist in Transportrichtung 6 spiegelsymmetrisch ausgestaltet. Dabei verläuft eine Symmetrieachse 16 senkrecht zur Transportrichtung 6 und, bezogen auf die Breite 14, zentral durch die Strahlungsquelle 8 hindurch. Im vorliegenden Ausführungsbeispiel verläuft die Symmetrieachse 16 somit zwischen den beiden Leuchtdiodenzeilen 9, 10. Die zwei Leuchtdiodenzeilen 9, 10 sind hier zentral in den Reflektor 11 integriert. Ein Abstand 17 zwischen den zwei Leuchtdiodenzeilen 9, 10 ist vorteilhafterweise kleiner oder gleich einem Abstand 32 der Leuchtdioden zu der in der Aufnahmeebene 7 liegenden Speicherleuchtstoffschicht 2. Die Reflektorflächen 12, 13 weisen auf ihren zur Speicherleuchtstoffschicht 2 hin gewandten Oberflächen reflektierende Schichten auf, die für von der Speicherleuchtstoffschicht 2 reflektiertes Löschlicht hochgradig reflektierend sind. Durch den Reflektor 11 wird Löschlicht, das von der Speicherleuchtstoffschicht 2 reflektiert oder gestreut wird, in Richtung der Speicherleuchtstoffschicht 2 zurückreflektiert. Durch diese Zurückreflexion besteht die Möglichkeit, dass das Löschlicht nun zum Löschen der Röntgeninformationen in die Speicherleuchtstoffschicht 2 eindringt.

Fig. 2 zeigt ein zweites Ausführungsbeispiel der Löschvorrichtung 1. Die Speicherleuchtstoffplatte ist hier nicht dargestellt. Dargestellt ist eine Auflage 18, oberhalb der sich die Aufnahmeebene 7 zum Aufnehmen und Führen der Speicherleuchtstoffplatte befindet. Die Löschvorrichtung 1 enthält den Reflektor 11. Dieser hat hier zwei Reflektorflächen 19 und 20, die parallel zu der Aufnahmeebene 7 und der Auflage 18 verlaufen. Die Reflektorflächen 19, 20 weisen jeweils eine Struktur auf, die hier im Wesentlichen dreieckförmig, nach Art von feinen Sägezähnen ausgestaltet ist. Mittels dieser Struktur ist ein retroreflektives Profil der Reflektorflächen 19, 20 realisiert.

Fig. 3 zeigt ein drittes Ausführungsbeispiel der Löschvorrichtung 1. In diesem dritten Ausführungsbeispiel ist der Reflektor 11 rinnen-oder trogförmig ausgestaltet. Die Querschnittsfläche des Reflektors 11 weist somit die Form einer Rinne auf. Dazu hat der Reflektor 11 zwei geradlinige Reflektorflächen 21, 22, die ausgehend von der Strahlungsquelle 8 mit stumpfen Innenwinkeln α schräg nach außen verlaufen. Die Innenwinkel betragen hier ca. 165°.

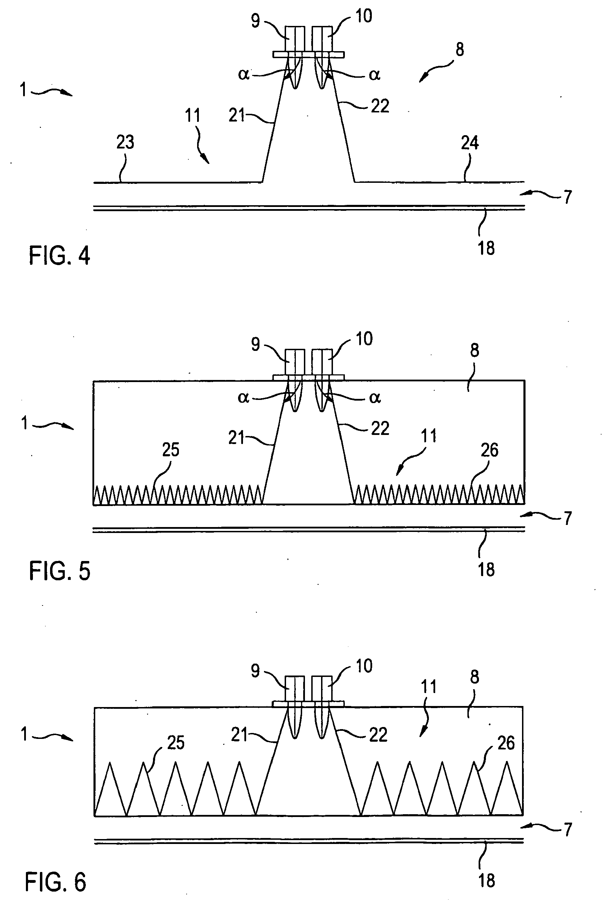

Fig. 4 zeigt ein viertes Ausführungsbeispiel der Löschvorrichtung 1. Der Reflektor 11 ist hier mittels der zwei geradlinigen Reflektorflächen 21, 22 ebenfalls rinnenförmig ausgestaltet. Die Innenwinkel α der rinnenförmigen Querschnittsfläche betragen hier ca. 110°. Der Reflektor 11 weist ausgehend von den unteren Enden der Rinne seitliche Reflektor-Fortsätze 23, 24 auf, die sich eben und parallel zu der Aufnahmeebene 7 für die Speicherleuchtstoffplatte nach außen erstrecken.

Fig. 5 zeigt ein fünftes Ausführungsbeispiel der Löschvorrichtung 1. Der Reflektor 11 ist hier, wie der Reflektor 11 gemäß dem vierten Ausführungsbeispiel, mittels der zwei geradlinigen Reflektorflächen 21, 22 rinnenförmig ausgestaltet. Die Innenwinkel α der rinnenförmigen Querschnittsfläche betragen ca. 110°. Der Reflektor 11 weist ausgehend von den unteren Enden der Rinne seitliche Reflektor-Fortsätze 25, 26 auf, die sich parallel zu der Aufnahmeebene 7 für die Speicherleuchtstoffplatte nach außen erstrecken. Die seitlichen Reflektor-Fortsätze 25, 26 haben hier eine feine dreieckförmige Struktur. Mittels dieser Struktur ist ein retroreflektives Profil der Reflektor-Fortsätze 25, 26 realisiert.

Fig. 6 zeigt ein sechstes Ausführungsbeispiel einer Löschvorrichtung 1. Der Reflektor 11 entspricht hier weitgehend dem Reflektor der Löschvorrichtung 1 gemäß dem fünften Ausführungsbeispiel nach Fig. 5. Allerdings haben die seitlichen Reflektor-Fortsätze 25, 26 hier eine grobe dreieckförmige Struktur. Mittels dieser Struktur ist ebenfalls ein retroreflektives Profil der Reflektor-Fortsätze 25, 26 realisiert.

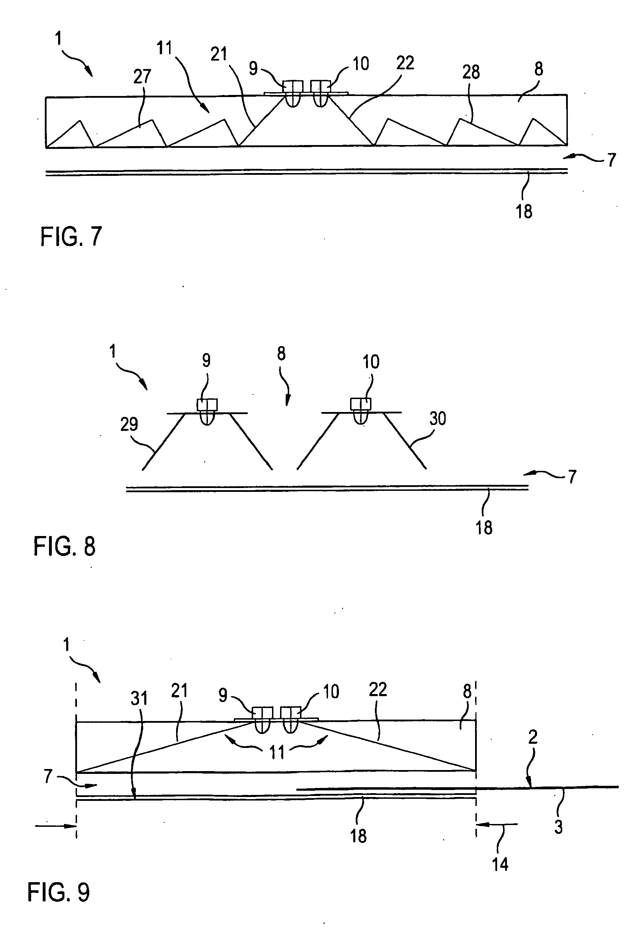

Fig. 7 zeigt ein siebtes Ausführungsbeispiel der Löschvorrichtung 1. Der Reflektor 11 ist hier mittels zweier geradlinigen Reflektorflächen 21, 22 ebenfalls rinnenförmig ausgestaltet. Allerdings sind die Reflektorflächen 21, 22 hier kürzer, als diejenigen der Ausführungsbeispiele gemäß der Fig. 4-6. Der Reflektor 11 weist ausgehend von den unteren Enden der Reflektorflächen 21, 22 der Rinne seitliche Reflektor-Fortsätze 27, 28 auf, die sich parallel zu der Aufnahmeebene 7 für die Speicherleuchtstoffplatte nach außen erstrecken. Die seitlichen Reflektor-Fortsätze 27, 28 haben hier eine grobe sägezahnförmige Struktur mit einer nur geringen Anzahl von Sägezähnen. Mittels dieser Struktur ist auch ein retroreflektives Profil der Reflektor-Fortsätze 27, 28 realisiert.

Fig. 8 zeigt ein achtes Ausführungsbeispiel der Löschvorrichtung 1. In diesem achten Ausführungsbeispiel enthält die Strahlungsquelle 8 die zwei Leuchtdiodenzeilen 9, 10, die hier allerdings jeweils einen eigenen Reflektor aufweisen. Die Leuchtdiodenzeile 9 ist in einen trogförmigen Reflektor 29 und die Leuchtdiodenzeile 10 in einen trogförmigen Reflektor 30 integriert. Die beiden Reflektoren 29, 30 sind voneinander getrennt, so dass das von den Leuchtdiodenzeilen 9, 10 ausgegebene Löschlicht voneinander getrennt auf die in der Löschvorrichtung befindliche Speicherleuchtstoffschicht auftreffen. Die Leuchtdiodenzeilen 9, 10 geben Löschlicht in verschiedenen Wellenlängenbereichen aus. Die Leuchtdiodenzeile 9 gibt Löschlicht im blauen Wellenlängenbereich und die Leuchtdiodenzeile 10 im roten Wellenlängenbereich aus. Dadurch lässt sich vorteilhafterweise eine gute "Farbtrennung", und damit eine hohe Löscheffizienz, erreichen. In Transportrichtung 6 der Speicherleuchtstoffplatte trifft daher zuerst blaues und anschließend rotes Löschlicht auf die Speicherleuchtstoffschicht. Ferner ist die Intensität des langwelligen, roten Löschlichts größer, als die Intensität des kurzwelligen, blauen Löschlichts. Die Intensitätsanteil des roten Löschlichts beträgt hier vorteilhafterweise ca. 66% und der Intensitätsanteil des blauen Löschlichts ca. 33%.

Zur Gewährleistung einer besonders effizienten Farbtrennung kann es von Vorteil sein, zumindest einen der Reflektoren 29 bzw. 30 in der Weise asymmetrisch auszugestalten, dass die dem jeweils anderen Reflektor 30 bzw. 29 zugewandte Flanke des jeweiligen Reflektortroges einen kleineren Innenwinkel aufweist als die dem jeweils anderen Reflektor 30 bzw. 29 abgewandte Flanke.

Fig. 9 zeigt die erfindungsgemäße Löschvorrichtung 1 gemäß dem dritten Ausführungsbeispiel nach Fig. 3. Im vorliegenden Ausführungsbeispiel weist die Löschvorrichtung 1 allerdings eine dem Reflektor 11, in Richtung senkrecht zur Transportgeschwindigkeit 6 betrachtet, gegenüberliegende weitere Reflexionsfläche 31 auf. Die Reflexionsfläche 31 ist zum Reflektieren von Löschlicht ausgestaltet, das von der Strahlungsquelle 8 ausgegeben wurde. Von der Reflexionsfläche 31 reflektiertes, weiteres Löschlicht wurde ferner gegebenenfalls bereits von der Speicherleuchtstoffschicht 2 und dem Reflektor 11 reflektiert. Zum Reflektieren von Löschlicht kann insbesondere auf der der Strahlungsquelle 8 zugewandten Seite der Auflage 18, d. h. auf der dem Reflektor 11 abgewandten Seite der Aufnahmeebene 7, die Reflexionsfläche 31 aufgebracht sein. Die Reflexionsfläche 31 kann vorteilhafterweise als dünne Schicht auf die Auflage 18 aufgebracht sein. Die Reflexionsfläche 31 ist somit derart angeordnet, dass die Speicherleuchtstoffplatte 3 zwischen dem Reflektor 11 und der Reflexionsfläche 31 hindurch transportiert werden kann. Die Reflexionsfläche 31 kann das Löschlicht gerichtet oder diffus zum Reflektor 11 reflektieren. Die Reflexionsfläche 31 ist hier in Transportrichtung 6 vorteilhafterweise so breit, wie der Reflektor 11. Die Fig. 9 zeigt die in die Löschvorrichtung 1 einfahrende Speicherleuchtstoffplatte 3. Die Reflexionsfläche 31 gewährleistet vorteilhafterweise, dass das von der Strahlungsquelle 8 ausgegebene Löschlicht auch dann mit einem hohen Wirkungsgrad zum Löschen beiträgt, wenn die Speicherleuchtstoffplatte 3 sich noch nicht vollständig in der Löschvorrichtung 1 befindet. Insbesondere wird gewährleistet, dass die führende Kante der Speicherleuchtstoffschicht 2 mit erhöhter Effizienz gelöscht wird. Ähnliches gilt, wenn die Speicherleuchtstoffplatte 3 aus der Löschvorrichtung 1 herausfährt. Auch beim Ausführungsbeispiel nach Fig. 9 gilt, dass es zum Löschen der Speicherleuchtstoffschicht 2 ebenso möglich ist, die Speicherleuchtstoffplatte 3 in der Löschvorrichtung 1 ruhen zu lassen und die Strahlungsquelle 8 mitsamt des Reflektors 11 und der gegenüberliegenden Reflexionsschicht 31 entlang der Speicherleuchtstoffplatte 3 zu transportieren.

Zur weiteren Verbesserung der Löscheffizienz ist es möglich, an der Strahlungsquelle 8, d. h. insbesondere an den Leuchtdiodenzeilen 9, 10 Zusatzoptiken vorzusehen, die den Emissionswinkel der Leuchtdioden verkleinern. Dadurch kann die auf der Speicherleuchtstoffschicht 2 angestrahlte Fläche schmäler ausgestaltet werden. Dies erhöht die Leistungsdichte zum Löschen.

## Patentansprüche

1. Vorrichtung (1) zum Löschen einer Speicherleuchtstoffschicht (2) mit
- einer Strahlungsquelle (8) zum Erzeugen und Aussenden einer Löschstrahlung,
- einem Antrieb (5) zum Erzeugen einer Relativbewegung zwischen der Speicherleuchtstoffschicht (2) und der Strahlungsquelle (8), wobei die Speicherleuchtstoffschicht (2) in einer Aufnahmeebene (7) liegt bzw. bewegt wird, und
- einem Reflektor (11) zum Reflektieren von Strahlung,
wobei der Reflektor (11) zum Reflektieren von von der Speicherleuchtstoffschicht (2) reflektierter Löschstrahlung in Richtung der Speicherleuchtstoffschicht (2) angeordnet und ausgestaltet ist, und
wobei der Reflektor (11) eine rinnen- oder trogförmige Querschnittsfläche aufweist;
**dadurch gekennzeichnet,**
- **dass** eine Ausbreitung (14) des Reflektors (11) in Richtung (6) der Relativbewegung wenigstens zehnmal so groß ist, wie ein kleinster Abstand (15) des Reflektors (11) von der Aufnahmeebene (7), und
- **dass** die rinnen- oder trogförmige Querschnittsfläche stumpfe Innenwinkel (α) aufweist, die insbesondere größer oder gleich 130° sind, und/oder dass sich, in Richtung (6) der Relativbewegung betrachtet, an die beiden Enden der Rinne parallel zur Aufnahmeebene (7) verlaufende Re- flektor-Fortsatzflächen (23, 24; 25, 26; 27, 28) anschließen.

2. Vorrichtung nach Anspruch 1, **dadurch gekennzeichnet, dass** der Reflektor (11) eine ebene Reflektorfläche (12, 13) aufweist, die parallel zur Aufnahmeebene (7) verläuft.

3. Vorrichtung nach Anspruch 1 oder 2, **dadurch gekennzeichnet, dass** der Reflektor (11) eine Reflektorfläche (19, 20; 25, 26; 27, 28) mit einer Struktur aufweist.

4. Vorrichtung nach Anspruch 3, **dadurch gekennzeichnet, dass** die strukturierte Reflektorfläche (19, 20; 25, 26; 27, 28) retroreflektiv ausgestaltet ist, so dass sie zumindest einen Teil der Löschstrahlung an Stellen der Speicherleuchtstoffschicht (2) zurückreflektiert, an denen sie von der Speicherleuchtstoffschicht (2) zuvor reflektiert wurde.

5. Vorrichtung nach einem der vorhergehenden Ansprüche, **dadurch gekennzeichnet, dass** der Reflektor (11) wenigstens zwei Reflektorflächen aufweist, so dass der Reflektor (11), in Richtung der Relativbewegung betrachtet, zu beiden Seiten der Strahlungsquelle (8) ausgebildet ist.

6. Vorrichtung nach Anspruch 3 oder 4, **dadurch gekennzeichnet, dass** die Reflektor-Fortsatzflächen (25, 26; 27, 28) Strukturen aufweisen.

7. Vorrichtung nach einem der vorhergehenden Ansprüche, **dadurch gekennzeichnet, dass** der Reflektor (11) an der Aufnahmeebene (7) zugewandten Reflektorflächen (11, 12; 19, 20; 21-28) reflektierende Schichten aufweist.

8. Vorrichtung nach einem der vorhergehenden Ansprüche, **dadurch gekennzeichnet, dass** der Reflektor (11) in Richtung (6) der Relativbewegung spiegelsymmetrisch ausgestaltet ist, wobei eine Symmetrieachse (16) senkrecht zur Richtung (6) der Relativbewegung und, in Richtung (6) der Relativbewegung betrachtet, zentral durch die Strahlungsquelle (8) hindurch verläuft.

9. Vorrichtung nach einem der vorhergehenden Ansprüche, **dadurch gekennzeichnet, dass** die Strahlungsquelle (8) wenigstens zwei senkrecht zur Richtung (6) der Relativbewegung und parallel zu der Aufnahmeebene (7) verlaufende Zeilen (9, 10) mit Leuchtdioden aufweist.

10. Vorrichtung nach Anspruch 9, **dadurch gekennzeichnet, dass** die wenigstens zwei Zeilen (9, 10) mit Leuchtdioden in den Reflektor (11) integriert sind und ein Abstand (17) zwischen den wenigstens zwei Zeilen (9, 10) und/oder ein Abstand zwischen den einzelnen Leuchtdioden innerhalb der jeweiligen Zeilen (9, 10) kleiner oder gleich einem Abstand (32) der Leuchtdioden zu der Aufnahmeebene (7) ist.

11. A Vorrichtung nach Anspruch 9, **dadurch gekennzeichnet, dass** den wenigstens zwei Zeilen (9, 10) mit Leuchtdioden jeweils ein eigener Reflektor (29, 30) zugeordnet ist und die Leuchtdioden der jeweiligen Zeilen (9, 10) eine Strahlung in einem anderen, insbesondere schmalbandigen, Wellenlängenbereich emittieren, als die Leuchtdioden der anderen Zeilen (9, 10).

12. Vorrichtung nach Anspruch 11, **dadurch gekennzeichnet, dass** die wenigstens zwei Zeilen (9, 10) mit Leuchtdioden in Richtung (6) der Relativbewegung so hintereinander angeordnet sind, dass beim Ausführen der Relativbewegung zum Löschen der Speicherleuchtstoffschicht (2) eine kurzwellige Löschstrahlung vor einer langwelligen Löschstrahlung auf die Speicherleuchtstoffschicht (2) trifft.

13. Vorrichtung nach Anspruch 11 oder 12, **dadurch gekennzeichnet, dass** eine Intensität der langwelligen Löschstrahlung größer ist, als eine intensität der kurzwelligen Löschstrahlung.

14. Vorrichtung nach einem der vorhergehenden Ansprüche, **dadurch gekennzeichnet, dass** eine dem Reflektor (11), in Richtung senkrecht zur Richtung (6) der Relativbewegung betrachtet, gegenüberliegende, weitere reflektierende Fläche (31) zum Reflektieren von Löschstrahlung vorhanden ist.

15. Vorrichtung nach Anspruch 14, **dadurch gekennzeichnet, dass** die weitere reflektierende Fläche (31) auf der dem Reflektor (11) abgewandten Seite der Aufnahmeebene (7) angeordnet ist.

16. Vorrichtung nach Anspruch 14 oder 15, **dadurch gekennzeichnet, dass** die weitere reflektierende Fläche (31) in Richtung (6) der Relativbewegung eine Ausbreitung (14) aufweist, die zumindest so groß ist, wie diejenige des Reflektors (11).

17. System mit einer Vorrichtung (1) nach einem der vorhergehenden Ansprüche und einer Speicherleuchtstoffschicht (2).

18. System nach Anspruch 17, **dadurch gekennzeichnet, dass** die Speicherleuchtstoffschicht (2) einen Reflexionsgrad für die Löschstrahlung von größer oder gleich 70%, insbesondere größer oder gleich 80%, aufweist.

## Claims

1. An apparatus (1) for erasing a storage phosphor layer (2) comprising
- a radiation source (8) for producing and emitting erasing radiation,
- a drive (5) for producing a relative movement between the storage phosphor layer (2) and the radiation source (8), the storage phosphor layer (2) lying or being moved in a receiving plane (7), and
- a reflector (11) for reflecting radiation,
the reflector (11) being disposed and formed for reflecting erasing radiation reflected by the storage phosphor layer (2) in the direction of the storage phosphor layer (2), and the reflector (11) having a groove- or trough-shaped cross-sectional area,
**characterised in**
- **that** a propagation (14) of the reflector (11) in the direction (6) of the relative movement is at least ten times as great as a smallest distance (15) between the reflector (11) and the receiving plane (7), and
- **that** the groove- or trough-shaped cross-sectional area has obtuse inner angles (α), which are in particular greater than or equal to 130°, and/or that, as observed in the direction (6) of the relative movement, reflector extension surfaces (23, 24; 25, 26; 27, 28) adjoin both ends of the groove parallel to the receiving plane (7).

2. The apparatus according to Claim 1, **characterised in that** the reflector (11) has a flat reflector surface (12, 13) which extends parallel to the receiving plane (7).

3. The apparatus according to Claim 1 or 2, **characterised in that** the reflector (11) has a reflector surface (19, 20; 25, 26; 27, 28) with a structure.

4. The apparatus according to Claim 3, **characterised in that** the structured reflector surface (19, 20; 26, 26; 27, 28) is retro-reflective in form so that it reflects back at least part of the erasing radiation to locations of the storage phosphor layer (2) where it was previously reflected by the storage phosphor layer (2).

5. The apparatus according to any of the preceding claims, **characterised in that** the reflector (11) has at least two reflector surfaces so that, as observed in the direction of the relative movement, the reflector (11) is formed on both sides of the radiation source (8).

6. The apparatus according to Claim 3 or 4, **characterised in that** the reflector extension surfaces (25, 26; 27, 28) have structures.

7. The apparatus according to any of the preceding claims, **characterised in that** the reflector (11) has reflective layers on reflector surfaces (11, 12; 19, 20; 21-28) facing towards the receiving plane (7).

8. The apparatus according to any of the preceding claims, **characterised in that** the reflector (11) is formed in a mirror image in the direction (6) of the relative movement, an axis of symmetry (16) extending perpendicularly to the direction (6) of the relative movement and, as considered in the direction (6) of the relative movement, centrally through the radiation source (8).

9. The apparatus according to any of the preceding claims, **characterised in that** the radiation source (8) has at least two lines (9, 10) with light-emitting diodes extending perpendicularly to the direction (6) of the relative movement and parallel to the receiving plane (7).

10. The apparatus according to Claim 9, **characterised in that** the at least two lines (9, 10) with light-emitting diodes are integrated into the reflector (11), and a distance (17) between the at least two lines (9, 10) and/or a distance between the individual light-emitting diodes within the respective lines (9, 10) is smaller than or equal to a distance (32) between the light-emitting diodes and the receiving plane (7).

11. The apparatus according to Claim 9, **characterised in that** a particular reflector (29, 30) is respectively assigned to the at least two lines (9, 10) with light-emitting diodes, and the light-emitting diodes of the respective lines (9, 10) emit radiation in a different, in particular narrow-band wavelength range than the light-emitting diodes of the other lines (9, 10).

12. The apparatus according to Claim 1, **characterised in that** the at least two lines (9, 10) with light-emitting diodes are disposed one behind the other in the direction (6) of the relative movement such that when implementing the relative movement in order to erase the storage phosphor layer (2) short-wave erasing radiation strikes the storage phosphor layer (2) before a long-wave erasing radiation.

13. The apparatus according to Claim 1 or 2, **characterised in that** an intensity of the long-wave erasing radiation is greater than an intensity of the short-wave erasing radiation.

14. The apparatus according to any of the preceding claims, **characterised in that**, as considered in the direction perpendicular to the direction (6) of the relative movement, a further reflective surface (31) opposite the reflector (11) is provided for reflecting the erasing radiation.

15. The apparatus according to Claim 4, **characterised in that** the further reflective surface (31) is disposed on the side of the receiving plane (7) facing away from the reflector (11).

16. The apparatus according to Claim 4 or 5, **characterised in that** the further reflective surface (31) has propagation in the direction (6) of the relative movement which is at least as great as that of the reflector (11).

17. A system with an apparatus (1) according to any of the preceding claims and a storage phosphor layer (2).

18. The system according to Claim 7, **characterised in that** the storage phosphor layer (2) has a degree of reflection for the erasing radiation of greater than or equal to 70%, in particular greater than or equal to 80%.

## Revendications

1. Dispositif (1) d'effacement d'une couche d'enregistrement luminescente (2) comportant :
- une source de rayonnement (8) destinée à produire et à émettre un rayonnement d'effacement,
- un dispositif d'entraînement (5) destiné à produire un mouvement relatif entre la couche d'enregistrement luminescente (2) et la source de rayonnement (8), la couche d'enregistrement luminescente (2) reposant ou pouvant être déplacée dans un plan de réception (7), et
- un réflecteur (11) destiné à réfléchir le rayonnement,
le réflecteur (11) étant disposé et conçu de manière à réfléchir le rayonnement d'effacement, renvoyé par la couche d'enregistrement luminescente (2), en direction de la couche d'enregistrement luminescente (2), et
le réflecteur (11) présentant une surface de section transversale en forme de rigole ou d'auge ;
**caractérisé en ce que**
- une étendue (14) du réflecteur (11) dans le sens (6) du mouvement relatif est au moins dix fois plus grande qu'un plus petit écart (15) existant entre le réflecteur (11) et le plan de réception (7), et **en ce que**
- la surface de section transversale en forme de rigole ou d'auge présente des angles intérieurs obtus (α), qui sont en particulier supérieurs ou égaux à 130°, et/ou **en ce que** des surfaces de prolongement de réflecteur (23, 24 ; 25, 26 ; 27, 28) se prolongeant parallèlement au plan de réception (7) font suite aux deux extrémités de la rigole, considéré dans le sens (6) du mouvement relatif.

2. Dispositif selon la revendication 1, **caractérisé en ce que** le réflecteur (11) présente une surface de réflecteur (12, 13) plane qui se prolonge parallèlement au plan de réception (7).

3. Dispositif selon la revendication 1 ou 2, **caractérisé en ce que** le réflecteur (11) présente une surface de réflecteur (19, 20 ; 25, 26 ; 27, 28) pourvue d'une structure.

4. Dispositif selon la revendication 3, **caractérisé en ce que** la surface de réflecteur (19, 20 ; 25, 26 ; 27, 28) structurée est conçue pour être rétroréfléchissante, de sorte qu'au moins une partie du rayonnement d'effacement soit rétroréfléchie en des emplacements de la couche d'enregistrement luminescente (2) où elle était précédemment réfléchie par la couche d'enregistrement luminescente (2).

5. Dispositif selon l'une des revendications précédentes, **caractérisé en ce que** le réflecteur (11) présente au moins deux surfaces de réflecteur de sorte que le réflecteur (11), considéré dans le sens du mouvement relatif, est conçu des deux côtés de la source de rayonnement (8).

6. Dispositif selon la revendication 3 ou 4, **caractérisé en ce que** les surfaces de prolongement de réflecteur (25, 26 ; 27, 28) présentent des structures.

7. Dispositif selon l'une des revendications précédentes, **caractérisé en ce que** le réflecteur (11) présente des couches réfléchissantes situées sur des surfaces de réflecteur (11, 12 ; 19, 20 ; 21-28) tournées vers le plan de réception (7).

8. Dispositif selon l'une des revendications précédentes, **caractérisé en ce que** le réflecteur (11) est de conception symétrique dans le sens (6) du mouvement relatif, un axe de symétrie (16) se prolongeant perpendiculairement au sens (6) du mouvement relatif en passant par le centre de la source de rayonnement (8), vu dans le sens (6) du mouvement relatif.

9. Dispositif selon l'une des revendications précédentes, **caractérisé en ce que** la source de rayonnement (8) présente au moins deux rangées (9, 10) de diodes électroluminescentes disposées perpendiculairement au sens (6) du mouvement relatif et parallèlement au plan de réception (7).

10. Dispositif selon la revendication 9, **caractérisé en ce que** les au moins deux rangées (9, 10) de diodes électroluminescentes sont intégrées dans le réflecteur (11) et **en ce qu'**un écart (17) existant entre les au moins deux rangées (9, 10) et/ou un écart existant entre les différentes diodes électroluminescentes au sein des rangées (9, 10) respectives est inférieur ou égal à un écart (32) existant entre les diodes électroluminescentes et le plan de réception (7).

11. Dispositif selon la revendication 9, **caractérisé en ce que** les au moins deux rangées (9, 10) de diodes électroluminescentes disposent chacune d'un réflecteur propre (29, 30) et les diodes électroluminescentes des rangées (9, 10) respectives émettent un rayonnement dans une plage de longueurs d'ondes différente, notamment à bande étroite, de celle des diodes électroluminescentes des autres rangées (9, 10).

12. Dispositif selon la revendication 11, **caractérisé en ce que** les au moins deux rangées (9, 10) de diodes électroluminescentes sont agencées les unes derrière les autres dans le sens (6) du mouvement relatif de sorte que la couche d'enregistrement luminescente (2) subissant le mouvement relatif destiné à effacer ladite couche d'enregistrement luminescente (2) soit tout d'abord soumise à un rayonnement d'effacement à ondes courtes puis à un rayonnement d'effacement à ondes longues.

13. Dispositif selon la revendication 11 ou 12, **caractérisé en ce qu'**une intensité du rayonnement d'effacement à ondes longues est plus élevée qu'une intensité du rayonnement d'effacement à ondes courtes.

14. Dispositif selon l'une des revendications précédentes, **caractérisé en ce qu'**il est prévu une surface réfléchissante supplémentaire (31) en regard du réflecteur (11), considéré dans la direction perpendiculaire au sens (6) du mouvement relatif, qui est destinée à réfléchir le rayonnement d'effacement.

15. Dispositif selon la revendication 14, **caractérisé en ce que** la surface réfléchissante supplémentaire (31) est agencée sur la face du plan de réception (7) qui est détournée du réflecteur (11).

16. Dispositif selon la revendication 14 ou 15, **caractérisé en ce que** la surface réfléchissante supplémentaire (31) présente, dans le sens (6) du mouvement relatif, une étendue (14) qui est au moins aussi importante que celle du réflecteur (11).

17. Système comportant un dispositif (1) selon l'une des revendications précédentes et une couche d'enregistrement luminescente (2).

18. Système selon la revendication 17, **caractérisé en ce que** la couche d'enregistrement luminescente (2) présente un degré de réflexion concernant le rayonnement d'effacement qui est supérieur ou égal à 70 %, notamment supérieur ou égal à 80 %.
